(19) Europäisches Patentamt
European Patent Office
Office européen des brevets

(11) Numéro de publication: **0 033 702**
**B1**

(12) **FASCICULE DE BREVET EUROPEEN**

(45) Date de publication du fascicule du brevet: **04.01.84**

(21) Numéro de dépôt: **81400156.6**

(22) Date de dépôt: **03.02.81**

(51) Int. Cl.³: **C 07 D 307/92,**
C 12 N 1/38, C 12 N 15/00
//C07C47/57, C07C47/575

(54) **Dérivés de nitro-2 naphtofurannes, leur préparation et leur application notamment comme régulateurs de la croissance cellulaire.**

(30) Priorité: **04.02.80 FR 8002354**

(43) Date de publication de la demande:
**12.08.81 Bulletin 81/32**

(45) Mention de la délivrance du brevet:
**04.01.84 Bulletin 84/1**

(84) Etats contractants désignés:
**AT BE CH DE FR GB IT LI LU NL SE**

(56) Documents cités:
**FR - A - 2 424 266**
**US - A - 4 128 659**

**EUROPEAN JOURNAL OF MEDICINAL CHEMISTRY CHIMICA THERAPEUTICA, vol. 13, no. 5, septembre-octobre 1978 PARIS (FR) R. ROYER et al.: "Recherches sur les dérivés nitrés d'intérèt biologique XIV. Activités contre les microorganismes des benzologues et furologues du nitro-2-benzofuranne", pages 407-409**

(73) Titulaire: **ANVAR Agence Nationale de Valorisation de la Recherche**
**43, rue Caumartin**
**F-75436 Paris Cedex 09 (FR)**

(72) Inventeur: **Royer, René Richard**
**12, rue du Pont des Loges**
**F-75007 Paris (FR)**
Inventeur: **Buisson, Jean-Pierre**
**8, rue de Strasbourg**
**Sartrouville F-78500 Yvelines (FR)**

(74) Mandataire: **Lepeudry-Gautherat, Thérèse et al,**
**Cabinet Armengaud Jeune Casanova, Akerman, Lepeudry 23 boulevard de Strasbourg**
**F-75010 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Courier Press, Leamington Spa, England.

Dérivés de nitro-2 naphtofurannes, leur préparation et leur application notamment comme régulateurs de la croissance cellulaire

La présente invention a pour objet des dérivés de nitro-2 naphtofurannes répondant à la formule

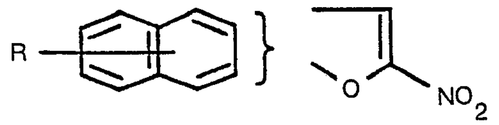

I

dans laquelle la liaison entre le cycle furanne et le noyau naphtalène se fait en positions (1,2), (2,3) ou (2,1) du naphtalène, et R, qui peut se trouver sur une position libre du naphtalène, représente un atome d'halogène ou un groupe méthoxy.

Les atomes d'halogène représentés par R sont en particulier le chlore et le brome.

Les composés de l'invention peuvent être préparés par condensation du bromonitrométhane et d'aldéhydes naphtaléniques ortho-hydroxylés (II), selon le schéma réactionnel suivant:

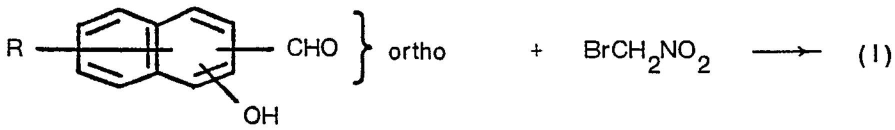


II

Dans la formule II R a la même signification que dans la formule I.

Ce type de condensation a été décrit à partir d'aldéhydes salicyliques par R. ROYER et coll. (Bull. Soc. Chim., 1970, *10* p 3740). Elle est effectuée en présence d'un accepteur d'acide. Etant donné que le aldéhydes ortho-hydroxylés II se prêtent plus ou moins bien à une telle condensation, il convenait d'en adapter les conditions pour chaque produit. Elle peut donc être effectuée en présence de carbonate de potassium, soit dans l'eau, soit dans l'acétone, à froid ou à chaud, ou bien en présence de morpholine selon la technique décrite par L. RENE et R. ROYER (Eur. J. Med. Chem., 1979, *14*, 471).

Les aldéhydes ortho-hydroxylés intermédiaires de formule II sont en partie décrits dans la littérature, mais la plupart d'entre eux ne sont guère accessibles par les procédés mentionnés. Les méthodes suivantes sont proposées:

— le formyl-1 hydroxy-2 méthoxy-3 naphtalène (II; R = $CH_3O$) peut être préparé à partir de méthoxy-3 naphtol-2, soit par réaction de Reimer et Tiemann, (cf. ROYER et coll., Bull. Soc. Chim., 1967, p 2405), soit par traitement avec le dichlorométhyl méthyl éther en présence de $TiCl_4$;

— les dérivés halogénés, en particulier le chlore-4 formyl-2 hydroxy-1 naphtalène (II; R = Cl décrit par W. J. BEGLEY et J. GRIMSHAW, J. Chem. Soc., Perkin Trans I. 1975, p 1840) et le bromo-6 formyl-1 hydroxy-2 naphtalène (II; R = Br, décrit par E. MUNDLOS et T. PAPENFUHS, brevet allemand No 2 305 071 du 14 août 1974) peuvent être préparés respectivement à partir du chloro-4 naphtol-1 et du bromo-6 naphtol-2 par traitement avec le dichlorométhyl méthyl éther;

— le formyl-1 hydroxy-2 méthoxy-6 naphtalène (II; R = $CH_3O$, décrit par S. V. KESSAR et coll., J. Indian. Chem., 1973, p 624), le formyl-1 hydroxy-2 méthoxy-7 naphtalène (II; R = $CH_3O$, décrit par R. ADAMS et coll., J. Amer. Chem. Soc., 1942, *64*, 1795), et le formyl-2 hydroxy-3 méthoxy-5 napthalène (II; R = $CH_3O$; composé nouveau), peuvent être préparés par déméthylation sélective, au moyen de chlorure d'aluminium dans le chlorure de méthylène, respectivement du diméthoxy-2,6 formyl-1 naphtalène (N. P. BUU—HOI et D. LAVIT, J. Chem. Soc., 1955, p 2776), du diméthoxy-2,7 formyl-1 naphtalène (BUU—HOI, idem) et du diméthoxy-3,5 formyl-2 naphtalène (R. A. BARNES et W. M. BUSCH, J. Amer. Chem. Soc., 1959, *81*, 4705).

Comme autre élément de l'art antérieur, on peut citer le document FR—A—2 424 266 qui est relatif à des dérivés nitrés de naphtofurannes ainsi qu'à leur préparation et à leur application comme médicaments. La liaison entre le noyau benzénique et le noyau benzo b-furanne se fait en positions (6,7), (4,5) ou (5,6). Ces dérivés sont préparés en effectuant la condensation du bromonitrométhane et d'aldéhydes ortho-hydroxylés naphtaléniques comme décrit dans la brevet français 2092891. Ils ont une activité anti-bactérienne étendue et peuvent être notamment utilisés comme agents anti-bactériens pour le traitement des infections à bactéries gram + et gram — et mixtes.

Les exemples suivants illustrent la préparation des composés de l'invention. Les exemples 1 et 2 concernent la préparation des composés intermédiaires (II) et l'exemple 3 la préparation des composés finals (I).

Tous les nouveaux composés décrits ici présentent des analyses centésimales pour C, H et N conformes à ± 0,2% à la théorie. Ils donnent des spectres de RMN $^1H$ compatibles avec leurs structures.

Exemple 1

Formylations des méthoxy-3 naphtol-2, chloro-4 naphtol-1 et bromo-6 naphtol-2.

Une solution constituée avec 2,1 moles de tétrachlorure de titane et 1,1 mole de dichlorométhyl méthyl éther dans 1 litre de chlorure de méthylène fraîchement rectifié est maintenue sont agitation, pendant 15 mn, à 0°C. On y ajoute ensuite, goutte à goutte, en maintenant la température en-dessous de 5°C, une quantité proportionnelle à 1 mole du naphtol en solution dans au moins 1 litre de chlorure de méthylène. On abandonne à 5°C pendant 2 h, laisse revenir à la température ambiante, verse dans de l'acide chlorhydrique dilué, épuise au chloroforme, filtre au besoin les résines, lave à l'eau, élimine le solvant par chauffage sous pression réduite et recristallise. On obtient ainsi respectivement:

(a) le formyl-1 hydroxy-2 méthoxy-3 naphtalène, F = 112°C (du cyclohexane); rendement 65%

(b) le chloro-4 formyl-2 hydroxy-1 naphtalène, F = 103°C (de l'éthanol); rendement 79%;

(c) le bromo-6 formyl-1 hydroxy-2 naphtalène, F = 152°C (du cyclohexane); rendement 90%.

Exemple 2

Déméthylations des diméthoxy formyl naphtalènes.

Ces réactions ont été effectuées avec une quantité double en poids de chlorure d'aluminium, dans 1 litre de chlorure de méthylène par mole d'éther traité. On maintient sous agitation à la température ambiante, pendant 15 mn, le chlorure d'aluminium dans le chlorure de méthylène puis ajoute lentement le diméthoxy formyl naphtalène en solution dans le minimum de chlorure de méthylène. On abandonne pendant 3 h sous agitation, verse dans de l'acide chlorhydrique dilué, extrait au chloroforme, lave la phase organique à l'eau et l'épuise à la soude ou à la potasse. Par neutralisation avec de l'acide chlorhydrique, l'extrait alcalin libère:

(d) le formyl-1 hydroxy-2 méthoxy-6 naphtalène à partir du diméthoxy-2,6 formyl-1 napthalène, F = 131—132°C (du toluène); rendement 80%;

(e) le formyl-1 hydroxy-2 méthoxy-7 naphtalène à partir du diméthoxy-2,7 formyl-1 naphtalène, F = 128—129°C (du toluène); rendement 87%;

(f) le formyl-2 hydroxy-3 méthoxy-5 naphtalène à partir du diméthoxy-3,5 formyl-2 naphtalène, F = 107°C (du cyclohexane); rendement 85%.

Exemple 3

Condensations des aldéhydes ortho hydroxylés (II) et du bromonitrométhane.

— TECHNIQUE A. (Dans l'acétone, à 0°C)

A un mélange constitué avec des quantités proportionnelles à 1 mole d'aldéhyde convenable (II) et 2 moles de carbonate de potassium dans 3 litres d'acétone pure, on ajoute goutte à goutte, en agitant vigoureusement et en maintenant la température à 0°C, une solution formée d'une quantité de bromonitrométhane correspondant à 2 moles, dans 500 ml d'acétone. On poursuit l'agitation à 0°C pendant 2 h, laisse revenir progressivement à la température ambiante, abandonne pendant 18 h, puis chauffe à l'ébullition, au reflux, pendant 2 h. On filtre, lave le résidue minéral à l'acétone et évapore le solvant de la phase organique. Le produit résultant est repris dans le chloroforme et chromatographié sur colonne d'alumine, avec élution par le même solvant. On termine par recristallisation dans le benzène ou l'alcool ou le mélange de ces deux solvants.

— TECHNIQUE B (Dans l'acétone à l'ébullition)

Le même mélange d'aldéhyde (II) dans l'acétone et de carbonate de potassium que précédemment est porté à l'ébullition, avant d'y ajouter goutte à goutte, le bromonitrométhane en solution acétonique. Après la fin de l'addition, le chauffage à l'ébullition est poursuivi pendant 4 h, avant le même traitement.

— TECHNIQUE C (Dans l'eau à 20°C)

On maintient pendant 1 h à la température ambiante, en agitant, des quantités proportionnelles à 1 mole d'aldéhyde hydroxylé (II) et 2 moles de carbonate de potassium dans 35 litres d'eau. On ajoute lentement la quantité correspondant à 1,5 mole de bromonitrométhane dans 300 ml d'éthanol et abandonne sous agitation pendant 48 h. On filtre le précipité, le lave à l'eau et le dissout dans du chloroforme. On achève comme dans les techniques précédentes.

— TECHNIQUE D (Dans l'eau à l'ébullition)

On chauffe pendant 1 h à l'ébullition, au reflux, la même solution aqueuse d'aldéhyde ortho hydroxylé (II) et de carbonate de potassium que ci-dessus. On y ajoute ensuite rapidement 1,5 mole de bromonitrométhane et poursuit le chauffage, en agitant, pendant 1 h. On laisse refroidir et traite selon l'usage.

— TECHNIQUE E (Avec de la morpholine)

On chauffe à l'ébullition, au reflux, dans un appareil muni d'un capteur d'eau, une solution à 10% dans le benzène d'un mélange de 1 mole d'aldéhyde ortho hydroxylé (II) et 2 moles de morpholine. Lorsque la quantité voulue d'eau est séparée, on refroidit extérieurement dans de la glace, ajoute en une seule fois 1,1 mole de bromonitrométhane puis abandonne pendant 15 h à la température ambiante et termine par 1 h de chauffage à l'ébullition. On lave soigneusement à l'eau, filtre sur une colonne d'alumine et achève l'élution au chloroforme.

Les rendements de ces diverses préparations, expérimentées pour chaque produit synthétisé, sont indiqués comparativement dans le tableau ci-dessous.

| Aldéhydes o-hydroxylés (II) | Nitro-2 naphtofurannes (I) | | | | | | |
|---|---|---|---|---|---|---|---|
| | N° | F°C | Rendement (%) selon la technique de préparation | | | | |
| | | | A | B | C | D | E |
| a | 1 | 180 | 25 | 21 | 44 | 34 | 50 |
| c | 2 | 250 | 21 | 17 | 43 | 47 | 27,5 |
| d | 3 | 188 | 66 | 30 | 8 | 55 | 10 |
| e | 4 | 200 | 65 | 28 | 13 | 42 | 75 |
| b | 5 | 165 | 25 | 16 | 16 | 8 | 72 |
| f | 6 | 199 | 4 | 4 | 0 | 0 | 62 |

Les formules développées et les nomenclatures des divers composés précédents sont les suivantes:

Composé No. 1:
nitro-2 méthoxy-9 naphto[2,1-b]-furanne

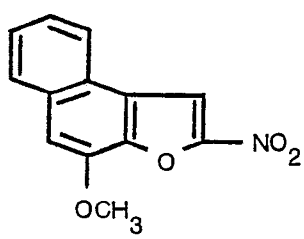

Composé No. 2:
nitro-2 bromo-6 naphto[2,1-b]-furanne

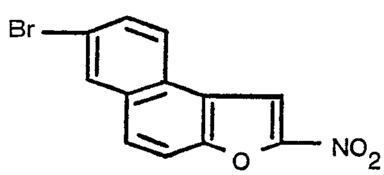

Composé No. 3:
nitro-2 méthoxy-6 naphto[2,1-b]-furanne

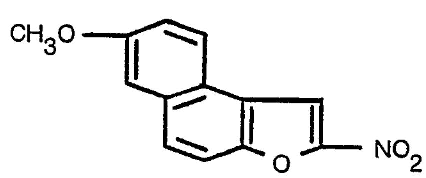

Composé No. 4:
nitro-2 méthoxy-5 naphto[2,1-b]-furanne

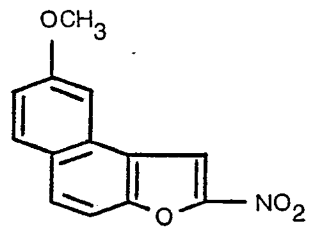

Composé No. 5:
nitro-2 chloro-5 naphto[1,2-b]-furanne

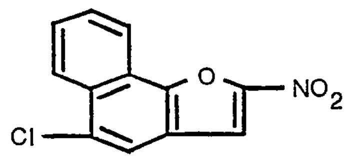

Composé No. 6:
nitro-2 méthoxy-8 naphto[2,3-b]-furanne

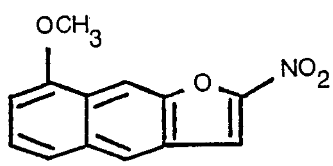

Les composés de l'invention ont été soumis à une expérimentation biologique sur des microorganismes.

Un pouvoir mutagène des composés a été détecté par test qualitatif sur des souches de Salmonella TA 1537, TA 1538 et TA 98 de la collection de l'Institut Pasteur. Ces essais ont été effectués avec ou sans activation et la mesure du pouvoir mutagène a montré qu'il est sensible à la présence du mélange d'activation qui agit dans le sens de la détoxification.

On a en particulier constaté que le composé No. 3 de l'invention possède un pouvoir mutagène 1000 fois plus élevé que le mutagène de référence l'ICR 191.

Au vu de ces résultats, les composés de l'invention sont utilisables comme réactifs de laboratoire, notamment comme régulateurs de la croissance cellulaire, par exemple comme mutagènes de référence.

## Revendications

1. Dérivés de nitro-2 naphtofurannes répondant à la formule

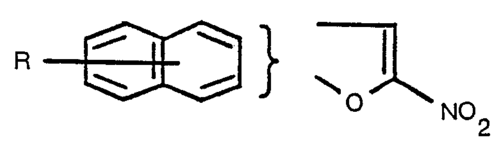

I

dans laquelle la liaison entre le cycle furanne et le noyau naphtalène se fait en positions (1,2), (2,3) ou (2,1) du naphtalène, et R, qui peut se trouver sur une position libre du naphtalène, représente un atome d'halogène ou un groupe méthoxy.

2. Composés selon la revendication 1, répondant à la formule I dans laquelle R est un groupe méthoxy.

3. Composés selon la revendication 1, répondant à la formule I dans laquelle R est un atome de chlore ou de brome.

4. Le nitro-2 méthoxy-6 naphto[2,1-b]-furanne.

5. Procédé de préparation des composés définis dans la revendication 1, procédé caractérisé en ce que l'on effectue la condensation du bromonitrométhane et d'aldéhydes naphtaléniques ortho-hydroxylés de formule

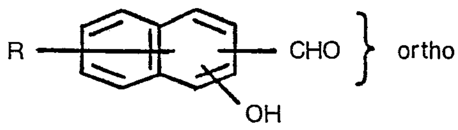

II.

6. Application des composés spécifiés dans l'une quelconque des revendications 1 à 4 comme régulateurs de la croissance cellulaire à l'exclusion de leur application dans des méthodes de traitement thérapeutique du corps humain ou animal ou des méthodes de diagnostic appliquées au corps humain ou animal.

**Patentansprüche**

1. 2-Nitronaphtofuranderivate, gemäß der Formel

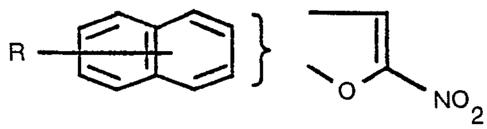

I

in der die Bindung zwischen dem Furan-Ring und dem Naphtalin-Kern in den Positionen (1, 2), (2, 3) oder (2, 1) des Naphtalins erfolgt und der an einer freien Positions des Naphtalins sitzende Substituent R ein Halogen-Atom oder eine Methoxy-Gruppe ist.

2. Verbindungen nach Anspruch 1 gemäß der Formel I, in denen der Substituent R eine Methoxy-Gruppe ist.

3. Verbindungen nach Anspruch 1 gemäß der Formel I, in denen der Substituent R ein Chlor- oder Brom-Atom ist.

4. 2-Nitro-6-methoxy-naphto-[2,1-b]-furan.

5. Verfahren zur Herstellung der Verbindungen nach Anspruch 1, dadurch gekennzeichnet, daß eine Kondensation von Bromnitromethan an ortho-hydroxylierten Naphtalin-Aldehyde der Formel

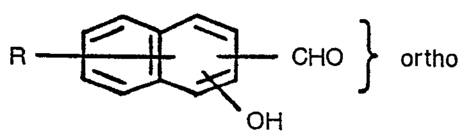

II.

durchgeführt wird.

6. Anwendung der Verbindungen nach einem der Ansprüche 1 bis 4 als Zellwachstumsregulatoren, ausgenommen die Anwendung dieser Verbindungen zur therapeutischen Behandlung des menschlichen oder tierischen Körpers oder in diagnostischen Verfahren am menschlichen oder tierischen Körper.

**Claims**

1. 2-Nitronaphthofuran derivatives corresponding to the formula

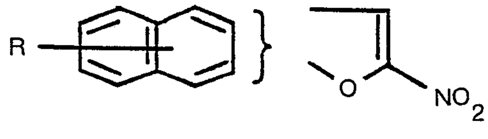

I

in which the linkage between the furan ring and the naphthalene nucleus is formed in the 1,2-, 2,3- or 2,1- positions of the naphthalene, and R, which can be located in any free position of the naphthalene, represents a halogen atom or a methoxy group.

2. Compounds according to Claim 1, corresponding to the formula I in which R is a methoxy group.

3. Compounds according to Claim 1, corresponding to the formula I in which R is a chlorine or bromine atom.

4. 2-Nitro-6-methoxynaphtho[2,1-b]furan.

5. Process for the preparation of the compounds defined in Claim 1, which process is characterized in that bromonitromethane is condensed with ortho-hydroxynaphthaldehydes of the formula

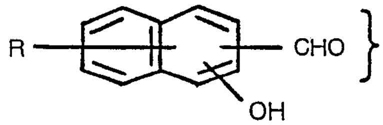 ortho II.

6. Application of the compounds specified in any one of Claims 1 to 4 as cell growth regulators, excluding their application in methods of therapeutic treatment of the human or animal body or diagnostic methods applied to the human or animal body.